# EUROPÄISCHE PATENTSCHRIFT

(11) **EP 1 035 181 B1**
(45) Veröffentlichungstag und Bekanntmachung des Hinweises auf die Patenterteilung: **28.05.2003**
(21) Anmeldenummer: 00105093.9
(22) Anmeldetag: 10.03.2000
(51) Int. Cl.: C09D 13/00, A61K 7/025, A61K 7/032

(54) **Pigmenthaltige Gelmasse auf Ölbasis**
Pigmented oil-based gel composition
Composition de gel pigmentée à base d'huile

(30) Priorität: 11.03.1999 DE 19910870
(43) Veröffentlichungstag der Anmeldung: 13.09.2000
(73) Patentinhaber: Schwan-STABILO Cosmetics GmbH & Co., 90562 Heroldsberg (DE)
(72) Erfinder:
(74) Vertreter: Leissler-Gerstl, Gabriele

(56) Entgegenhaltungen:
- WO-A-95/31961
- US-A- 4 425 328
- US-A- 5 340 386
- PATENT ABSTRACTS OF JAPAN vol. 017, no. 462 (C-1101), 24. August 1993 (1993-08-24) & JP 05 112424 A (KAO CORP), 7. Mai 1993 (1993-05-07)

## Beschreibung

Die Erfindung betrifft pigmenthaltige Gelmassen auf Ölbasis, diese enthaltende Farbstifte und ein Verfahren zur Herstellung von Farbstiften.

Pigmenthaltige Gelmassen auf Ölbasis sind disperse Systeme, die aus einem Gerüst der gelbildenden Komponente mit darin eingelagerter Ölkomponente bestehen. Die Gelmasse enthält außerdem Pigmente zur Anfärbung. Derartige Massen sind für das Auftragen von Farben in vielen Bereichen geeignet. Als Hauptanwendungsgebiete für pigmentierte Gelmassen kommen Farbmaterialien zum Zeichnen und Malen und das Gebiet der dekorativen Kosmetik inbetracht, wo sie in vielen Formen, unter anderem für Make-up, cremige Lidschatten, Lippenstifte, Eyeliner- und Augenbrauenstifte anwendbar sind. Insbesondere können derartige pigmenthaltige Gelmassen zur Herstellung von Kosmetikstiften verwendet werden.

Zum Zeichnen, Malen oder Schminken verwendete Materialien sollen leicht aufgetragen werden können, nach dem Auftragen aber gut haltbar sein und möglichst wasserfest, im Fall von Kosmetikstiften auch tränenfest, sowie transferresistent sein, d.h. nicht auf andere Oberflächen und Gegenstände abfärben und nicht aus dem Auftragsbereich ausbluten.

Bekannte Farbstiftrezepturen basieren auf einem Gemisch aus Wachsen und Ölen, das mit Pigmenten angefärbt wird. So werden beispielsweise für Lippen- oder Lidschattenstifte aus natürlichen oder mineralischen Wachsen, natürlichen oder synthetischen Ölen oder Mineralölen und üblichen Zusatzstoffen, wie Lanolin oder Lanolinderivaten aufgebaute Stiftformulierungen beschrieben. In der Regel stellen diese Zubereitungen thixotrope Systeme dar, die sich unter den beim Auftragen auftretenden Scherkräften verflüssigen und so ein sanftes Auftragen ermöglichen.

Im Bereich der Kosmetik wurden gelartige Mischungen bisher überwiegend für Deodorant- und Antiperspirantstifte eingesetzt. Sie enthalten einen erheblichen Anteil an flüchtigen Siliconölen, wie Cyclomethicone oder Dimethicone, oder Kohlenwasserstoffölen, wie Isoparaffin. Diese Gele haben den Vorteil, daß sie sich gut auftragen lassen, aber den Nachteil, daß sie mechanisch nicht belastbar sind. Für Deodorantstifte überwiegt der Vorteil der leichten Auftragbarkeit, der Stabilität kommt aufgrund der Form dieser Stifte keine so große Bedeutung zu. Deodorantstifte haben im Verhältnis zu ihrer Länge einen großen Durchmesser und die Anforderungen an die Festigkeit sind aufgrund des Aufbaus der Stifte nicht sehr hoch. Es wurde gefunden, daß sich Massen, die als Deodorantstifte gut geeignet sind, nur bedingt zu dünneren Minen formen lassen und insbesondere, da die Masse zu weich und unstrukturiert ist, nur mit großen Schwierigkeiten oder garnicht mehr entformen lassen.

Weiterhin wurde versucht, Oleogele mit Candelillawachs herzustellen und zu Minen zu formen. Candelillawachs ist aufgrund seines Glanzes für Lippenstifte und Lidschattenstifte beliebt. Es wurde jedoch gefunden, daß die bekannten Oleogele ein zu geringes Ölbindevermögen aufweisen, sodaß es zu einer Phasentrennung kommt und das in der Masse enthaltene Öl in die Umgebung wandert. Dies beeinträchtigt die ästhetische Erscheinung des Stiftes. Außerdem verlieren durch das Ausölen die Stifte ihre Elastizität und werden brüchig. Darüberhinaus wurde gefunden, daß durch das Wegdiffundieren, Verdampfen oder Auswandern des Öls die aus der Masse hergestellten Minen so stark schrumpfen, daß bei einem Versuch zur Herstellung von Farbstiften in Holz eingelegte Minen nach kurzer Zeit aus den Holzhülsen herausrutschten.

Es war nun Aufgabe der vorliegenden Erfindung, die oben beschriebenen Nachteile zu beseitigen und eine Gelmasse auf Ölbasis bereitzustellen, die ein hohes Ölbindevermögen aufweist, die geeignet ist, um zu Minen für Stifte, insbesondere Kosmetikstifte, verarbeitet zu werden. Weiterhin war es Aufgabe der Erfindung, eine Masse bereitzustellen, deren Textur stabil ist, die möglichst wasser- und tränenfest ist und transferresistent ist, d.h. nach dem Auftragen nicht auf Oberflächen oder Gegenstände abfärbt. Außerdem sollen die Applikationseigenschaften der Masse gut sein, d.h. sie soll bis zu etwa 40°C auftragbar sein, ohne sich zu verformen oder zu verschmieren, aber auch nicht zu hart sein. Für Kosmetikstifte ist es außerdem wünschenswert, daß die Gelmasse nur aus wenigen, kosmetisch annehmbaren Komponenten aufgebaut ist. Darüberhinaus soll die Masse durch Gießen oder Extrudieren geformt werden können.

Diese Aufgaben werden gelöst mit der erfindungsgemäßen pigmentierten Gelmasse auf Ölbasis, die 0,1 bis 25 Gew.-% einer Hydroxyfettsäure mit 10 bis 20 C-Atomen, 0,1 bis 30 Gew.-% C₃₀₋₄₅-Alkylmethicone, 1 bis 70 Gew.-% einer Ölkomponente und 0,1 bis 50 Gew.-% Pigmente enthält.

Überraschenderweise wurde gefunden, daß eine Kombination aus einer Hydroxyfettsäure mit C₃₀₋₄₅-Alkylmethicone eine hervorragende Matrix für die Bindung von Ölkomponenten bildet, und eine Gelmasse mit ausgezeichneten Eigenschaften liefert, die sich durch Extrusion und Gießen sehr gut formen läßt. Die vier Hauptbestandteile liefern eine strukturierte Masse, die auch bei thermischer Belastung bis zu 45°C keine Phasentrennung zeigt. C₃₀₋₄₅-Alkylmethicone bildet zusammen mit der Hydroxyfettsäure eine stabile Matrix, die das Öl und die Pigmente dauerhaft und fest bindet. Darüberhinaus wird der Masse eine ausgezeichnete Temperaturstabilität vermittelt. Diese erlaubt es, die Masse bei erhöhten Temperaturen zu verarbeiten und ohne Kühlung zu lagern.

Ein erfindungswesentlicher Bestandteil der Gelmasse ist die Hydroxyfettsäure, die gelbildend an dem Strukturaufbau wirkt. Es ist eine Fettsäure mit 10 bis 20 C-Atomen, die mindestens eine Hydroxygruppe, bevorzugt in der Kette und nicht endständig, trägt. Bevorzugt ist es eine C₁₆-Hydroxyfettsäure, und besonders bevorzugt wird 12-Hydroxystearinsäure aufgrund ihrer guten Verfügbarkeit eingesetzt. Die Hydroxyfettsäure ist in der Masse in einem Anteil von 0,1 bis 25 Gew.-%, bezogen auf die aus Pigment, Methicone, Öl und Fettsäure gebildete Masse, enthalten. Unter 0,1 Gew.-% ist eine Wirkung nicht mehr nachweisbar. Ein Anteil von mehr als 25 Gew.-% läßt den Anteil der anderen strukturbildenden Komponenten so weit absinken, daß ein haltbarer Strukturaufbau schwierig ist. Bevorzugt wird die Hydroxyfettsäure in einer Menge von 1 bis 20 Gew.-% eingesetzt. Besonders gute Ergebnisse werden mit einem Anteil von 2 bis 15 Gew.-% erhalten.

Die andere für die Struktur wichtige Komponente ist C₃₀₋₄₅-Alkylmethicone. "C₃₀₋₄₅-Alkylmethicone" ist der INCI-Name für ein Siliconharz mit langkettigen Alkylresten, das wachsartige Eigenschaften aufweist und unter dieser Bezeichnung im Handel erhältlich ist. Diese Komponente trägt wesentlich zu den mit der erfindungsgemäßen Gelmasse erzielten Vorteilen bei. Es wurde gefunden, daß bei Verwendung anderer Siliconharze, z.B. Stearyl-Dimethicone, oder natürlicher Wachse, z.B. Candellilawachs, das Ölbindevermögen nicht ausreicht, um Ölkomponenten in einer stabilen Struktur zu halten, auch nicht in Kombination mit einer Hydroxyfettsäure. C₃₀₋₄₅-Alkylmethicone ist in der erfindungsgemäßen Gelmasse in einem Anteil von 0,1 bis 30 Gew.-% vorhanden. Bei einer Menge unter 0,1 Gew.-% können die vorteilhaften Eigenschaften nicht erzielt werden. Der Zusatz von mehr als 30 Gew.-% führt zu harten, brüchigen Massen mit unerwünschten Auftragseigenschaften. Bevorzugt wird das C₃₀₋₄₅-Alkylmethicone in einer Menge von 1 bis 30 Gew.-% eingesetzt, wobei besonders gute Ergebnisse mit einem Anteil von 5 bis 20 Gew.-% erzielt werden.

Ein weiterer wesentlicher Inhaltsstoff der erfindungsgemäßen Gelmasse ist die Ölkomponente. Die Ölkomponente kann pflanzliches, tierisches, mineralisches oder synthetisches Fett und/oder Öl und Wachs umfassen. So sind für die vorliegende Erfindung unter anderen Öle, Fette, fette Öle, Paraffine und Vaseline geeignet. All diese Komponenten werden von der erfindungsgemäßen Kombination aus Hydroxyfettsäure und C₃₀₋₄₅-Alkylmethicone gebunden. Je viskoser die Ölkomponente ist, desto geringer sollte die eingesetzte Menge sein, um bei der fertigen Zusammensetzung die gewünschten Eigenschaften zu erreichen. Als Beispiele für die Ölkomponente können natürliche und synthetische Öle, Mineralöle, Lanolin und Lanolinderivate, Isoparaffine, nichtflüchtige oder flüchtige Siliconöle, wie Cyclomethicone oder Dimethicone, und deren Mischungen genannt werden. Als fette Öle werden insbesondere pflanzliche Öle, z.B. hydrierte pflanzliche Öle eingesetzt.

Das Öl wird in einem Anteil von 1 bis 70 Gew.-% verwendet. Unter 1 Gew.-% wird die Masse zu viskos. Wenn der Anteil des Öls zu hoch wird, d.h. über 70% liegt, kann es zu einem Ausölen kommen, das unerwünscht ist. Die gewünschte Viskosität der Masse kann durch die Auswahl von Art und Menge der Ölkomponente mit wenigen Routineversuchen in geeigneter Weise eingestellt werden.

Ein Teil der Ölkomponente kann auch durch Wachs gebildet werden. Hierzu kommen natürliche und mineralische Wachse aber auch synthetische Wachse, z.B. Siliconwachse, inbetracht. Bevorzugt werden als Ölkomponente Mischungen aus ölartigen und wachsartigen Substanzen eingesetzt. Wenn die Gelmasse zur Herstellung von Kosmetikstiften verwendet werden soll, wird bevorzugt ein in der Kosmetik übliches Wachs verwendet, wie Bienenwachs, Carnaubawachs, Candelillawachs, Japanwachs, Montanwachs, mikrokristallines Wachs oder eine Mischung dieser Wachse. Besonders bevorzugt für eine Gelmasse, die zur Herstellung von dekorativer Kosmetik vorgesehen ist, ist Candelillawachs, wegen seines Glanzes beim Auftragen. Als Ölanteil werden hierfür bevorzugt flüchtige und/oder nichtflüchtige Öle eingesetzt. Besonders bevorzugt ist eine Mischung flüchtiger Siliconöle, wie Cyclomethicone oder Dimethicone, mit Wachsen, Paraffinen oder fetten Ölen. Die flüchtigen Siliconöle erleichtern einerseits das Auftragen der Gelmasse und erhöhen andererseits die Haltbarkeit und Wasserfestigkeit der aufgetragenen Schicht deutlich, wobei bei geeigneter Auswahl der "wind-burn effect" vermieden wird.

Der Anteil von Wachs wird abhängig von der Viskosität der Gelmasse und den gewünschten Viskositätseigenschaften des fertigen Produkts ausgewählt. Bevorzugt beträgt der Anteil an Wachs in der gesamten Zusammensetzung 0,1 bis 25 Gew.-%.

Der vierte erfindungswesentliche Bestandteil der Gelmasse ist ein Pigment oder eine Pigmentmischung. Hier werden die üblicherweise für pigmentierte Massen eingesetzten Substanzen verwendet. Beispiele für geeignete Pigmente sind Eisenoxide, Titandioxid, Glimmer, Talkum, Farblacke und Mischungen davon. Weiterhin können als Glanzpigmente plättchenförmige Metallpulver oder Glitter, z.B. in Form farbig beschichteter Aluminiumplättchen oder PET-Folien, enthalten sein. Bevorzugt werden die für dekorative Kosmetik verwendeten Pigmente eingesetzt. Der Gehalt des Pigmentes oder der Pigmentmischung in der erfindungsgemäßen Gelmasse liegt in einem Bereich von 0,1 bis 50 Gew.-%, bevorzugt 1 bis 40 Gew.-%, je nach der Farbintensität des verwendeten Pigmentes. Besonders bevorzugt ist eine Menge von 5 bis 30 Gew.-%.

Die erfindungsgemäße Gelmasse kann zusätzlich zu den aufgeführten Komponenten noch weitere Zusatz- und Hilfsstoffe enthalten, die für derartige Massen üblicherweise verwendet werden. Beispiele sind Füllstoffe, Verdickungsmittel, Feuchthaltemittel, Emulgatoren, Parfums, Aromastoffe, Antioxidantien und Konservierungsmittel, die in den üblicherweise verwendeten Mengen eingesetzt werden können.

Bevorzugt ist die erfindungsgemäße Gelmasse wasserfrei, sodaß Konservierungsmittel nicht unbedingt notwendig sind, da Mikroorganismen unter diesen Bedingungen nicht wachsen können. Die Verwendung von Antioxidantien ist empfehlenswert, insbesondere wenn die Ölkomponente ungesättigte Fettsäuren enthält, um ein Ranzigwerden oder Verderben der Masse zu verhindern.

Die Gesamtmenge der Zusatzstoffe sollte 45 Gew.-% nicht übersteigen und bevorzugt zusammen mit der Menge der Pigmente in einem Bereich von 0,1 bis 50 Gew.-%, bevorzugt 1 bis 40 Gew.-% und besonders bevorzugt in einem Bereicht von 5 bis 35 Gew.-% liegen.

Die erfindungsgemäße pigmentierte Gelmasse auf Ölbasis kann in beliebige Formen gebracht werden. Besonders bevorzugt wird sie zu Minen geformt und dann in Form von Stiften verwendet. Ein weiterer Vorteil der erfindungsgemäßen Gelmasse besteht darin, daß sie mit Gieß- und Extrusionsverfahren geformt werden kann. Besonders bevorzugt wird die erfindungsgemäße Gelmasse zu Farbstiften und Kosmetikstiften verarbeitet. Die hergestellten Minen mit einer Geltextur haben den Vorteil, daß sie sich gut auftragen lassen, ohne zu verformen oder zu schmieren.

Ein weiterer Gegenstand der vorliegenden Erfindung ist ein Farbstift, der eine Hülse aus Holz oder Kunststoff mit einer darin eingebetteten Mine aus einer pigmentierten Gelmasse auf Ölbasis umfaßt. Bevorzugt ist der Farbstift ein Kosmetikstift und besonders bevorzugt ist es ein Lidschatten-, Lippenkonturen-, Eyeliner-, Khol- oder Augenbrauenstift.

Der erfindungsgemäße Farbstift wird hergestellt, indem die erfindungsgemäß hergestellte pigmentierte Gelmasse geschmolzen wird und in einen Hülsenrohling eingegossen wird oder indem die pigmentierte Gelmasse extrudiert wird und der erhaltene Formling in einen Hülsenrohling eingelegt oder eingesetzt wird.

Die aus der erfindungsgemäß pigmentierten Gelmasse auf Ölbasis hergestellten Minen haben aufgrund des verbesserten Ölbindevermögens und einer hohen Temperaturstabilität positive Eigenschaften. Sie können bis zu 40°C angewendet werden. Die Minen haben eine ausreichende Härte und Elastizität und lassen sich gut spitzen.

Ein weiterer Gegenstand der Erfindung ist ein Verfahren zur Herstellung von Stiften unter Verwendung der erfindungsgemäßen Gelmasse wie es in den Ansprüchen 18 und 19 definiert ist.

Aufgrund der Stabilität der Gelmasse, kann diese ohne Probleme zu Minen, auch sehr dünnen Minen verarbeitet werden, z.B. aufgeschmolzen und geformt werden. Da einerseits die Gelmasse temperaturstabil ist und andererseits die geformten Minen eine ausreichende Festigkeit aufweisen, können die Stifte sowohl hergestellt werden, indem die erfindungsgemäße Gelmasse direkt in Hülsenrohlingeoder in auf eine Drehmechanik aufgesetzte Gießformen gegossen wird, die dann in an sich bekannter Weise zu Stiften weiterverarbeitet werden, oder aber es können zuerst in einem Arbeitsschritt Minen durch Gießen oder Extrusion hergestellt werden, und diese dann in die gewünschte Hülse oder in eine Drehmechanik eingesetzt werden. Beide Varianten bieten Vorteile und es kann für den jeweiligen Zweck die am besten geeignete Ausführungsform ausgewählt werden.

Gemäß einer Ausführungsform wird zur Herstellung von Minen die pigmentierte Gelmasse bevorzugt durch Gießen oder Extrusion geformt. Bevorzugt ist ein Formen mit Gießverfahren, da in diesem Fall die Masse direkt in den zur Aufnahme der Mine vorgesehenen Hülsenrohling gegossen werden kann, ohne nochmals entformt und weiterverarbeitet werden zu müssen. Der Rohling wird dann in an sich bekannter Weise zu einem Minenstift weiterverarbeitet. Auf diese Weise kann der Ausschuß gering gehalten werden.

In einer anderen bevorzugten Ausführungsform wird aus der erfindungsgemäß pigmentierten Gelmasse eine Mine durch Extrusion oder Gießen geformt und nach dem Entformen in eine Drehmechanik eingesetzt. Die erfindungsgemäße Gelmasse hat auch ohne Abstützung eine so stabile Struktur, daß sie in einer Drehmechanik heraus und herein gedreht werden kann, ohne abzubrechen. Sie eignet sich daher auch sehr gut für die Herstellung von Drehstiften.

Die Erfindung wird durch die folgenden Beispiele erläutert. In den Beispielen sind alle Mengenangaben in Gewichtsteilen aufgeführt.

### Beispiel 1 - Eyeliner

Aus den folgenden Komponenten wurde eine Gelmasse hergestellt (die Rohstoffe sind mit INCI-Bezeichungen angegeben):

| | |
|---|---|
| Pigmente | 30,000 |
| Caprylic Capric Triglyceride | 42,000 |
| Butyl Stearate | 5,000 |
| C₃₀₋₄₅-Alkyl Methicone | 10,000 |
| 12-Hydroxystearic Acid | 10,000 |
| Hydrogenated Vegetable Oil | 3,000 |

Alle Bestandteile, außer den Pigmenten, wurden auf 90°C erwärmt und geschmolzen und dann der geschmolzenen Masse die Pigmente zugesetzt. Anschließend wurde die Masse homogenisiert, entlüftet und bei 90°C in vorbereitete Hülsenrohlinge eingegossen und erkalten gelassen. Die erhaltenen Stifte wurden nach üblichen Verfahren weiterverarbeitet. Die erhaltenen Eyeliner ließen sich gut auftragen und lieferten eine wasser- und tränenfeste Beschichtung.

### Beispiel 2 - Lidschatten

Aus den folgenden Komponenten wurde eine Gelmasse hergestellt:

| | |
|---|---|
| Pigmente | 19,500 |
| Vegetable Oil | 20,000 |
| Jojoba Oil | 7,000 |

### Cyclomethicone 30,500

| | |
|---|---|
| C₃₀₋₄₅-Alkyl Methicone | 13,500 |
| 12-Hydroxystearic Acid | 7,500 |
| Candelilla Wax | 2,000 |

Alle Bestandteile außer Pigmenten und Cyclomethicone wurden auf 80°C erwärmt und geschmolzen und dann der geschmolzenen Masse die Pigmente zugesetzt. Dann wurde die Masse homogenisiert und entlüftet. Anschließend wurde das Cyclomethicone zugesetzt und gut eingemischt. Danach wurde die Masse bei 80°C in vorbereitete Hülsenrohlinge eingegossen und erkalten gelassen. Die erhaltenen Stifte ließen sich sehr gut auftragen und lieferten eine glänzende, abriebfeste Beschichtung.

### Beispiel 3 - Lipliner

Aus den folgenden Bestandteilen wurde eine Gelmasse hergestellt:

| | |
|---|---|
| Pigmente | 20,000 |
| Caprylic Capric Triglyceride | 30,000 |
| Hydrogenated Vegetable Oil | 12,000 |
| C₃₀₋₄₅-Alkyl-Methicone | 10,000 |
| 12-Hyroxystearic Acid | 12,000 |
| Mikrocrystalline Wax | 3,000 |
| Castor Oil | 13,000 |

Alle Bestandteile außer den Pigmenten wurden auf 80°C erwärmt und geschmolzen und dann wurden die Pigmente der geschmolzenen Masse zugesetzt. Die Masse wurde homogenisiert und entlüftet. Anschließend wurde die Masse bei 80°C in geeignete Formen eingegossen und abgekühlt. Die geformten Minen wurden entformt und in Hülsenrohlinge eingesetzt, geleimt und in üblicher Weise weiterverarbeitet. Die erhaltenen Stifte ließen sich gut spitzen und die erhaltenen Stifte eigneten sich gut für einen konturengenauen Auftrag, der haltbar und farbintensiv war.

## Patentansprüche

1. Pigmenthaltige Gelmasse auf Ölbasis enthaltend 0,1 bis 25 Gew.-% einer Hydroxyfettsäure mit 10 bis 20 C-Atomen, 0,1 bis 30 Gew.-% C₃₀₋₄₅-Alkylmethicone, 1 bis 70 Gew.-% einer Ölkomponente und 0,1 bis 50 Gew.-% Pigmente.

2. Gelmasse nach Anspruch 1, **dadurch gekennzeichnet, daß** die Hydroxyfettsäure 12-Hydroxystearinsäure ist.

3. Gelmasse nach Anspruch 1 oder Anspruch 2, **dadurch gekennzeichnet, daß** sie 0,5 bis 20 Gew.-%, bevorzugt 1 bis 15 Gew.-%, Hydroxyfettsäure enthält.

4. Gelmasse nach einem der vorhergehenden Ansprüche, **dadurch gekennzeichnet, daß** sie 1 bis 25 Gew.-%, bevorzugt 5 bis 20 Gew.-%, C₃₀₋₄₅-Alkylmethicone enthält.

5. Gelmasse nach einem der vorhergehenden Ansprüche, **dadurch gekennzeichnet, daß** die Ölkomponente pflanzliches, tierisches, mineralisches oder synthetisches Fett und/oder Öl, nichtflüchtiges oder flüchtiges Siliconöl und Wachs umfaßt.

6. Gelmasse nach einem der vorhergehenden Ansprüche, **dadurch gekennzeichnet, daß** sie Wachs in einen Anteil von 0,1 bis 25 Gew.-%, bezogen auf das Gewicht der Zusammensetzung, enthält.

7. Gelmasse nach Anspruch 6, **dadurch gekennzeichnet, daß** als Wachs natürliches, mineralisches oder synthetisches Wachs enthalten ist.

8. Gelmasse nach einem der vorhergehenden Ansprüche, **dadurch gekennzeichnet, daß** sie als Ölkomponente eine Mischung aus Öl und Wachs enthält.

9. Gelmasse nach einem der Ansprüche 7 oder 8, **dadurch gekennzeichnet, daß** als Wachs Bienenwachs, Carnaubawachs, Candelillawachs, Japanwachs, Montanwachs, mikrokristallines Wachs oder deren Mischung enthalten ist.

10. Gelmasse nach einem der vorhergehenden Ansprüche, **dadurch gekennzeichnet, daß** sie zusätzlich Füllstoffe enthält in einem Anteil von 0,1 bis 45 Gew.-% bezogen auf das Gewicht der Zusammensetzung.

11. Gelmasse nach einem der vorhergehenden Ansprüche, **dadurch gekennzeichnet, daß** sie Pigmente in einer Menge von 1 bis 40 Gew.-%, bevorzugt 5 bis 30 Gew.-%, enthält.

12. Gelmasse nach einem der vorhergehenden Ansprüche, **dadurch gekennzeichnet, daß** als Pigmente Eisenoxide, Titandioxid, Glimmer, Talkum, Farblacke oder Mischungen davon und/oder Glanzpigmente enthalten sind.

13. Gelmasse nach einem der vorhergehenden Ansprüche, **dadurch gekennzeichnet, daß** sie in Form einer gegossenen oder extrudierten Mine vorliegt.

14. Farbstift umfassend eine Mine aus einer Gelmasse nach einem der Ansprüche 1 bis 13, die von einem Hülsenrohling aus Holz oder Kunststoff umgeben ist.

15. Farbstift umfassend eine Mine aus einer Gelmasse nach einem der Ansprüche 1 bis 13, die in die Drehmechanik eines Drehstiftes eingesetzt ist.

16. Farbstift nach einem der Ansprüche 14 oder 15, **dadurch gekennzeichnet, daß** es ein Kosmetikstift ist.

17. Farbstift nach einem der Ansprüche 14 oder 15, **dadurch gekennzeichnet, daß** es ein Lidschatten-, Eyeliner-, Khol-, Augenbrauen- oder Lippenkonturenstift ist.

18. Verfahren zur Herstellung eines Farbstiftes, **dadurch gekennzeichnet, daß** eine Gelmasse nach einem der Ansprüche 1 bis 13 aufgeschmolzen wird und anschließend in einen Hülsenrohling für einen Minenstift aus Holz oder Kunststoff gegossen wird und dann zu einem Stift verarbeitet wird.

19. Verfahren zur Herstellung eines Farbstiftes, **dadurch gekennzeichnet, daß** zur Herstellung eines Drehstiftes eine Gelmasse nach einem der Ansprüche 1 bis 13 geschmolzen wird und entweder in eine auf eine Drehmechanik aufgesetzte Form gegossen wird oder in eine Gießform gegossen wird und die gebildete Mine nach dem Entformen in die Drehmechanik eines Drehstifts eingesetzt wird.

## Claims

1. Oil-based pigment-bearing gel material containing 0.1 to 25% by weight of a hydroxy fatty acid with 10 to 20 C-atoms, 0.1 to 30% by weight of C₃₀₋₄₅-alkyl methicone, 1 to 70% by weight of an oil component and 0.1 to 50% by weight of pigments.

2. A gel material according to claim 1 **characterised in that** the hydroxy fatty acid is 12-hydroxy stearic acid.

3. A gel material according to claim 1 or claim 2 **characterised in that** it contains 0.5 to 20% by weight, preferably 1 to 15% by weight, of hydroxy fatty acid.

4. A gel material according to one of the preceding claims **characterised in that** it contains 1 to 25% by weight, preferably 5 to 20% by weight, of C₃₀₋₄₅-alkyl methicone.

5. A gel material according to one of the preceding claims **characterised in that** the oil component includes vegetable, animal, mineral or synthetic fat and/or oil, non-volatile or volatile silicone oil and wax.

6. A gel material according to one of the preceding claims **characterised in that** it contains wax in a proportion of 0.1 to 25% by weight, with respect to the weight of the composition.

7. A gel material according to claim 6 **characterised in that** natural, mineral or synthetic wax is contained as the wax.

8. A gel material according to one of the preceding claims **characterised in that** as the oil component it contains a mixture of oil and wax.

9. A gel material according to one of claims 7 and 8 **characterised in that** as wax it contains beeswax, carnauba wax, candelilla wax, Japan wax, lignite wax, microcrystalline wax or a mixture thereof.

10. A gel material according to one of the preceding claims **characterised in that** it additionally contains fillers in a proportion of 0.1 to 45% by weight with respect to the weight of the composition.

11. A gel material according to one of the preceding claims **characterised in that** it contains pigments in an amount of 1 to 40% by weight, preferably 5 to 30% by weight.

12. A gel material according to one of the preceding claims **characterised in that** as pigments it contains iron oxides, titanium dioxide, mica, talcum, coloured lacquer or mixtures thereof and/or sheen pigments.

13. A gel material according to one of the preceding claims **characterised in that** it is in the form of a cast or extruded lead.

14. A colour pencil including a lead comprising a gel material according to one of claims 1 to 13 which is surrounded by a sheath blank of wood or plastic material.

15. A colour pencil including a lead comprising a gel material according to one of claims 1 to 13 which is fitted into the rotary mechanism of a rotary pencil.

16. A colour pencil according to one of claims 14 and 15 **characterised in that** it is a cosmetic pencil.

17. A colour pencil according to one of claims 14 and 15 **characterised in that** it is an eyeshadow, eyeliner, kohl, eyebrow or lipliner pencil.

18. A process for the production of a colour pencil **characterised in that** a gel material according to one of claims 1 to 13 is melted and then cast into a sheath blank for a lead-bearing pencil consisting of wood or plastic material and then processed to form a pencil.

19. A process for the production of a colour pencil **characterised in that** to produce a rotary pencil a gel material according to one of claims 1 to 13 is melted and either is poured into a mould fitted on to a rotary mechanism or poured into a casting mould and the lead formed after removal from the mould is fitted into the rotary mechanism of a rotary pencil.

## Revendications

1. Composition de gel pigmentée à base d'huile contenant 0,1 à 25 % en masse d'un hydroxyacide gras comportant 10 à 20 atomes de carbone, 0,1 à 30 % en masse d'une alkyl(en C₃₀ à C₄₅)-méthicone, 1 à 70 % en masse d'un composant huileux et 0,1 à 50 % en masse de pigments.

2. Composition de gel selon la revendication 1, **caractérisée en ce que** l'hydroxyacide gras est de l'acide 12-hydroxystéarique.

3. Composition de gel selon la revendication 1 ou la revendication 2, **caractérisée en ce qu'**elle contient 0,5 à 20 % en masse, de préférence 1 à 15 % en masse, d'hydroxyacide gras.

4. Composition de gel selon l'une des, revendications précédentes, **caractérisée en ce qu'**elle contient 1 à 25 % en masse, de préférence 5 à 20 % en masse, d'alkyl(en C₃₀ à C₄₅)-méthicone.

5. Composition de gel selon l'une des revendications précédentes, **caractérisée en ce que** la composante huileuse comprend de la graisse et/ou de l'huile végétale, animale, minérale ou synthétique, une huile de silicone non volatile ou volatile et de la cire.

6. Composition de gel selon l'une des revendications précédentes, **caractérisée en ce que** la cire est présente selon une proportion comprise entre 0,1 et 25 % en masse, sur la base de la masse de la composition.

7. Composition de gel selon la revendication 6, **caractérisée en ce qu'**elle contient, en tant que cire, de la cire naturelle, minérale ou synthétique.

8. Composition de gel selon l'une des revendications précédentes, **caractérisée en ce qu'**elle contient en tant que composante huileuse un mélange d'huile et de cire.

9. Composition de gel selon l'une des revendications 7 ou 8, **caractérisée en ce qu'**elle contient, en tant que cire, de la cire d'abeille, de la cire de carnauba, de la cire de Candellila, de la cire du Japon, de la cire de lignite, de la cire microcristalline ou leurs mélanges.

10. Composition de gel selon l'une des revendications précédentes, **caractérisée en ce qu'**elle contient, en outre, des matières de charge selon une proportion comprise entre 0,1 et 45 % en masse, sur la base de la masse de la composition.

11. Composition de gel selon l'une des revendications précédentes, **caractérisée en ce qu'**elle contient des pigments dans une quantité comprise entre 1 et 40 % en masse, de préférence entre 5 et 30 % en masse.

12. Composition de gel selon l'une des revendications précédentes, **caractérisée en ce qu'**elle contient en tant que pigments des oxydes de fer, du dioxyde de titane, du mica, du talc, des laques colorées ou leurs mélanges et/ou des pigments brillants.

13. Composition de gel selon l'une des revendications précédentes, **caractérisée en ce qu'**elle se présente sous la forme d'une mine coulée ou extrudée.

14. Crayon de couleur comprenant une mine composée d'une composition de gel selon l'une des revendications 1 à 13, qui est entourée d'une ébauche tubulaire en bois ou en matière synthétique.

15. Crayon de couleur comprenant une mine composée d'une composition de gel selon l'une des revendications 1 à 13, qui est utilisée dans un mécanisme de pivotement d'un crayon à fermeture par rotation.

16. Crayon de couleur selon l'une des revendications 14 ou 15, **caractérisé en ce qu'**il s'agit d'un crayon cosmétique.

17. Crayon de couleur selon l'une des revendications 14 ou 15, **caractérisé en ce qu'**il s'agit d'un crayon à paupières, d'un crayon à paupières, d'un eye-liner, d'un khôl, d'un crayon à sourcils ou d'un crayon pour le contour des lèvres.

18. Procédé de fabrication d'un crayon de couleur, **caractérisé en ce qu'**une composition de gel selon l'une des revendications 1 à 13 est fondue, puis coulée dans une ébauche tubulaire pour crayon à mine en bois ou en matière synthétique, puis façonnée en un crayon.

19. Procédé de fabrication d'un crayon de couleur, **caractérisé en ce que**, pour fabriquer un crayon à fermeture par rotation, on fait fondre une composition de gel selon l'une des revendications 1 à 13 puis, soit on la coule dans un moule posé sur un mécanisme de pivotement, soit on la moule dans un moule de coulée, et la mine formée est montée, après démoulage, dans le mécanisme de pivotement d'un crayon à fermeture par rotation.
